# NEW EUROPEAN PATENT SPECIFICATION

(11) **EP 1 593 747 B2**
(45) Date of publication and mention of the opposition decision: **15.05.2013**
(45) Mention of the grant of the patent: 23.12.2009
(21) Application number: 05252012.9
(22) Date of filing: 31.03.2005
(51) Int. Cl.: C12Q 1/68

(54) **Process for the enumeration and identification of microorganisms**
Verfahren zur Zählung und Identifikation von Mikroorganismen
Procédé d'énumeration et d'identification de micro-organismes

(30) Priority: 04.05.2004 US 567953 P
(43) Date of publication of application: 09.11.2005
(73) Proprietor: EMD Millipore Corporation, Billerica, MA 01821 (US)
(72) Inventor: Young, Barbara, Lexington, Massachusetts 02420 (US); Sage, Andrew, Littleton, Massachusetts 01460 (US)
(74) Representative: Kirkham, Nicholas Andrew

(56) References cited:
- WO-A-00/71675
- WO-A-01/59157
- WO-A-99/33559
- WO-A-99/60005
- WO-A-03/085109
- WO-A1-94/21780
- US-A- 5 766 868
- US-A1- 20060 286 557
- REINEKE K F ET AL: "DIRECT ENUMERATION OF ENTERIC BACTERIA IN FOOD, BEVERAGES AND WATER BY IN SITU HYBRIDIZATION TO 16S RIBOSOMAL RNA" ABSTRACTS OF THE GENERAL MEETING OF THE AMERICAN SOCIETY FOR MICROBIOLOGY, THE SOCIETY, WASHINGTON, DC, US, vol. 99, no. 16, 30 May 1999 (1999-05-30), page 516, XP008003668 ISSN: 1060-2011

## Description

The present invention relates to a process for enumerating and identifying microorganisms. More particularly, it relates to a process for amplifying specific components of the microbes present and then enumerating them on a membrane device in a single step with minimal incubation time based on detection of the amplified component product. Additionally, the microbes present may be identified by this method.

### BACKGROUND OF THE INVENTION

The traditional method of determining the presence or absence of microorganisms in liquids such as water, food products such as juices or pharmaceuticals has been to filter the fluid through a suitable membrane to trap any microorganisms present in the fluid on the surface of the membrane. The membrane is then placed on a growth media plate such as a Petri Dish filled with agar or other suitable media and then incubated for several days to allow colonies to develop from the captured organisms.

The plates are then removed and examined visually so that the number of colonies present can be counted. If the number is high enough, further tests may be conducted to determine exactly what organisms are present. (Often the mere presence of organisms is not in and of itself an indication that the fluid is unsafe and further work to identify the specific organisms is required.).

This process can typically span from two to fourteen days or more depending upon the organisms, their prescribed incubation time and growth rate and the additional identification tests that need to be run as well as when a sample was taken during the work day and when it will be ready on the next available work day.

Recently, new tests for the detection and enumeration of microbial contaminants have helped reduce the time required to conduct the tests to about 24 hours and even sometimes less. For example, ATP bioluminescence is a biochemical reaction that produces light energy as a product; this method has been used to detect and enumerate microbes in 1/3 the time of growth needed for the visual detection on media. Other technologies utilize fluorescent molecules, or other stains to rapidly detect microorganisms from a variety of samples on membranes. Probe hybridization technology has also been used to detect and enumerate microorganisms on membranes in shorter time frames than growth to colonies; in these cases, microbes that had been grown on a membrane for a short time are hybridized with nucleic acid probes and treated with a detection reagent to detect any micro-colonies present on the membrane surface.

US 2003-0003540-A1 improves upon the ATP tests. It captures microbe on a membrane and incubates the organisms on a media for several hours to a day. An enumeration test is first conducted (generally a bioluminescent ATP detection) and then PNA probes are used in a second test on the same sample to determine the identification of the microbes found.

None of these tests however allow one to have a test that detects, enumerates and identifies the microorganisms in real time (within a few hours) with the same accuracy and specificity of the classic multiday visual test. The present invention provides a single test that detects and enumerates the microorganism in a matter of a few hours on a membrane surface, and also allows the possible identification as well in the same procedure.

We are aware of International patent publication WO 99/60005 (FSM Technologies Limited) which describes methods and a filter device for purifying nucleic acids from whole cells in a whole blood sample. Cross-flow filtration is used to rupture cellular and nuclear membranes whereby the sample is passed across the surface of a porous membrane in a filter device, the path from the inlet to the outlet of the device being partially occluded allowing flow of cellular debris around the membrane to prevent it from clogging the pores.

International patent publication WO 01/59157 (Millipore Corp ) describes a test for the detection, enumeration and identification of microorganisms in a fluid sample. The test uses a filter through which a fluid sample is passed and onto which organisms within the fluid are deposited. It is then incubated on a growth medium and thereafter subjected to a presence absence test for organisms using an ATP/bioluminescence test. The same sample and filter is then subjected to a PNA probe assay for targeted organisms. Optionally, the locations of the organisms detected by each test can be compared with each other to determine the possibility of false positives and their elimination from the test results

International patent publication WO 03/085109 (Genesystems) relates to a method for extracting nucleic acids of microorganisms contained in a complex mixture, particularly a food mixture, comprising at least the following steps: the mixture is clarified; the microorganisms are retained on a filtering cartridge; said microorganisms are lysed; and the nucleic acids are recovered, concentrated, and purified. Also described is a filtering cartridge and an apparatus used for carrying out the extraction method, and a device for extracting, detecting, and if necessary quantifying the nucleic acids of microorganisms contained in a complex mixture.

### SUMMARY OF THE INVENTION

Essential and optional features of the present invention are set out in the accompanying main- and sub-claims respectively. Thus the present invention relates to a process for the detection, enumeration and identification of microorganisms in a fluid using membrane filtration, targeted amplification and detection such as by fluorescent detection or colorimetric detection. A key attribute of the present process is that the amplification step allows for the rapid detection, enumeration and identification of organisms that are present. The amplification targets a select molecule in the organism such as its DNA or RNA and increases the presence of that molecule to a level that is detectable by any number of available detection technologies in order to confirm that the target molecule is present. The amplification reagent contains one or more detection agents to indicate the presence of a target molecule(s) and allow one to differentiate between selected targets on a species or genus or even phylum, kingdom level.

### IN THE DRAWINGS

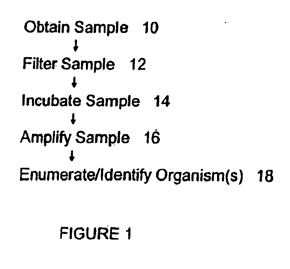

Figure 1 shows a block diagram of a preferred embodiment of the present invention.

### DETAILED DESCRIPTION OF THE SPECIFICATION

The present invention relates to a process for the rapid detection, enumeration and identification of microorganisms in fluids or soluble solids such as powders, salts, creams, tablets, etc. Such fluids include but are not limited to water, potable or otherwise, dairy such as milk, beer, wine, soft drinks, fruit juices, pharmaceuticals, parenterals, bacterial air counts, etc.

Current rapid technology allows one to detect and enumerate microorganisms in far less time than the classic methods of growth to visible colonies on agar or membrane filters. However, those organisms detected remain unidentified. This idea addresses the need for an immediate detection and enumeration system, and allows the possible identification of the detected organisms with specific probes at species or genus grouping in the amplification reactions, or at higher levels with phyla, or class specific probes.

The product visualized is a membrane-associated, targeted amplification reaction such as, but not inclusive of, the polymerase chain reaction (PCR) based method coupled to a membrane composed of hydrophilic domains bound by hydrophobic gridding such as is used in the MicroStar^{™} system available from Millipore Corporation of Billerica, Massachusetts. There are several components: a hydrophilic membrane covered with a hydrophobic gridding, reagents to carry out the targeted amplification consisting of nucleic acid, or peptide nucleic acid primers, enzymes, buffers and detergents, a fluorescent detection probe built into the amplification cocktail, and a mylar or plastic sleeve in which the reaction would be performed (chemoluminescent or chromogenic based detection systems may also be employed). The devices required are a temperature control system to maintain the temperature appropriate for targeted amplification reactions; for example, a temperature cycler would be needed for PCR reaction. Also, a detection device to count hydrophilic wells on the gridded membrane exhibiting a positive signal is required. The detection device could be as simple as a handheld UV light, or as complex as a MicroStar device.

The product can be used in the following manner. A sample containing microorganisms would be filtered through the gridded membrane and contaminant organisms captured in contrast to the cell rupturing cross-flow filtration method referred to in the above mentioned WO 99/60005. After rinsing, the amplification reagents would be sprayed onto the membrane surface, and the membrane would be placed into the Mylar® film sleeve and sealed. The sealed sleeve with the membrane and reagents would then be placed in device to control the environment of the amplification reaction. For example, for a PCR reaction, this would consist of a temperature cycler. After completion, the sample would be placed into the detection device. The device would detect those wells containing a positive signal produced by the fluorescent detection probe, or some like reagent. A positive signal would be consistent with the presence of a targeted microbial contaminant. Tests could be specific for microorganisms at the species, genus or group level inclusive of bacteria, yeast and mold, viruses, and protozoa. For example, the test could target the nucleic acid of the bacterial species *Pseudomonas aeruginosa*, all true pseudomonads, or broadly targeting all gram-negative bacteria, or all bacteria. This would allow one to obtain a measure of different types of organisms in the test sample, and determine the identity of any particular microbes of interest to the user.

Figure 1 shows a preferred process according to the present invention. In this process, as the first step 10, a sample in fluid form is obtained from the product to be diagnosed. For example, in environmental sampling, such as a water supply, a sample of water from the river, well or reservoir is obtained. For a food or pharmaceutical product, a sample is taken from the stock (typically this is the finished stock although it need not be so). If in liquid form it is simply used as is or may, if necessary, due to viscosity be diluted with deionized water. If in solid form, it is dissolved or dispersed in a suitable liquid, typically water or an alcohol.

It is then filtered through a membrane filter in the second step 12. The filter is then treated in step 16 with an amplification agent that contains one or more selected detector agents. The filter is then placed into an amplification device, such a thermal cycler for PCR amplification, for a time sufficient to amplify the sample to a level that can be detected or enumerated in step 18 either through a mechanical or electrical device or visually.

An incubation step 14 is included before the amplification step 16 so as to allow a micro-colony to develop from the individual retained microbes thus increasing the number of organisms available for amplification. Following the amplification step, the organisms are enumerated and/or identified in step 18.

A preferred device for holding the filter is a MILLIFLEX^{™} filter funnel having a 50 mm diameter and a 100 ml capacity, available from Millipore Corporation of Billerica, Massachusetts. Other devices such as the Steritest^{™} or Sterifil® filtration units available from Millipore Corporation of Billerica, Massachusetts that have hydrophobic grids formed on and through a hydrophilic membrane added may also be used, or membrane holders such as glass or stainless steel filter holders or funnels. Such devices are well known and available from a variety of sources including Millipore Corporation of Billerica, Massachusetts and Fisher Scientific, Inc, of Pittsburgh, Pennsylvania.

A preferred membrane is a MicroStar^{™} filter, available from Millipore Corporation of Billerica, Massachusetts. This filter is a 0.45 nominal pore size PVDF filter having a series of hydrophilic compartments separated by hydrophobic partitions that extend through the entire depth of the filter. Other filters that may be useful in this invention would also be hydrophilic and contain some type of hydrophobic partitioning.

As discussed in relation to the embodiment of Figure 1 above, the filtered sample is subject to an incubation step 14. Preferably, it is accomplished by placing the filter in contact with a growth media such as various agars or liquid media. The incubation is a short period of time, from 1 to 4 hours. The incubation allows the single microbes captured on the membrane to divide into a greater number of organisms for amplification making detection faster and easier, especially when the target in the organisms is difficult to amplify.

The filter is subjected to the amplification step for a short period of time, typically from 1 to 5 hours. The length of time depends upon the accuracy needed by the test, the rate of amplification for the amplification technology selected, the detection agent(s) selected, the method for detecting the agent (mechanical or human eye), the type of organism to be detected, the desired speed of the test vs. the accuracy of the test results and other such factors.

Suitable methods for amplification include nucleic acid based systems such as polymerase chain reaction (PCR) amplification in which a DNA or RNA sequence in the contaminating cell is amplified by an amplification agent. The PCR is typically conducted by placing the substrate (in this case a filter) containing the organism to be amplified, and having been treated with amplification agent during cycling to elevated temperatures for a period of time from about 30 minutes to 90 minutes in a thermal cycler. Commercially available kits for conducting a PCR amplification step are available from Applied Biosystems, Inc. or Roche Diagnostics. Thermal cyclers are available from these same companies.

Other possible amplification technologies include but are not limited to nucleic acid sequence based amplification (NASBA) available from Biomerieux, strand displacement amplification (SDA) available from Becton Dickenson, or linked linear amplification (LLA) available from Biorad. Also see EP 1407050A2.

It should be understood that the assays run herein with amplification step are on micro-colonies of microorganisms that develop from the single cells captured on the membranes, during the incubation step, i.e. one is enumerating the numbers of single organisms in a test sample by detection of targeted molecules that have been amplified from those same single organisms rather than detecting the individual organisms directly.

After a suitable time period for the filter in the amplification step, the filter is removed and subjected to an enumeration step 18.

A preferred enumeration test is by fluorescence detection of the amplified target. The target molecule is then detected through the detector agent incorporated into the amplification reagent such as a fluorescent tag, a radioactive tag, a colorimetric tag and the like. The agent is then read visually or digitally through an imagining device such as CCD camera with image processor. One such system is sold as MicroStar^{™} system, available from Millipore Corporation of Billerica, Massachusetts. By whatever means that is used, the count and location of the microbial colonies detected can be made.

During the enumeration test, the sample can also subjected to an identification test for the target organisms. They may be designed so as to specifically hybridize only to a single species or to an entire genus. The detector agents contain a tag such as an enzyme, hapten, fluorophore or radioisotope to indicate their presence in the sample. Such agents are available from a variety of sources including Sigma Genosys, and may consist of molecular beacons, dual labeled fluorescent probes, or fluorescent labeled probes to describe fluorescent probes, though chemiluminescent, colorimetric, or radiological detection systems may be used as well.

One may select one or more of such agents to detect one or more types of organisms. Some exist for individual organisms such as *E. coli* or *Salmonella sp*., *L. brevis*, etc, while others are more universal and simply detect the presence of the genus of the bacteria or whether the organism that has been captured is simply a bacteria, a yeast or fungi. Depending upon agent selected, one can use X-ray film, a fluorescent, or colorimetric detection or other such device to detect the presence of the tag of the agents which have been amplified with the target molecule such as the DNA or RNA of the organism and thereby identify the number and type of each target organism present.

## Claims

1. A process for detecting microorganism contamination in a liquid sample selected from water, food products and pharmaceuticals and enumerating and identifying any such microorganisms comprising the sequential steps of:
a) filtering the liquid sample through a membrane suitable for the retention of microorganisms to retain the microorganisms from said sample on said membrane;
c) subjecting the membrane to an amplification step wherein the amplification step contains one or more detection agents, wherein the amplification step is selected from the group consisting of polymerase chain reaction (PCR), nucleic acid sequence based amplification (NASBA), strand displacement amplification (SDA) and linked linear amplification (LLA); and
d) detecting the presence/absence of one or more microorganisms, the number detected and type,
**characterized by**, between steps (a) and (c), the step of
b) incubating the membrane for from 1 to 4 hours to increase the number of microorganisms retained on said membrane,
wherein the membrane is subjected to amplification in step c) for a period of 1 to 5 hours.

2. The process of claim 1 wherein the liquid sample is from 50 to 1000 milliliters.

3. The process of claim 1 or 2, wherein the membrane is selected from the group consisting of PVDF membrane having hydrophilic areas separated by hydrophobic partitions.

4. The process of any preceding claim, wherein the amplification is a nucleic acid-based amplification system.

5. The process of claim 4, wherein
a) the detection agent is selected from the group consisting of a fluorescent-labelled nucleic acid, peptide nucleic acid probe and mixtures thereof and is read by digital imaging system, or
b) the amplification is a nucleic acid-based amplification system in the form of polymerase chain reaction (PCR) and the detection agent is selected from the group consisting of a fluorescent-labelled nucleic acid, peptide nucleic acid probe and mixtures thereof and is read by digital imaging system, or
c) the reagent is selected from the group consisting of a DNA target and a RNA target and the detection agent is selected from the group consisting of a fluorescent-labelled nucleic acid, peptide nucleic acid probe and mixtures thereof and is read by digital imaging system, or
d) the amplification is a nucleic acid-based amplification system in the form of polymerase chain reaction (PCR), the reagent being selected from the group consisting of a DNA target and a RNA target and the detection agent is selected from the group consisting of a fluorescent-labelled nucleic acid, peptide nucleic acid probe and mixtures thereof and is read by digital imaging system.

6. The process of any preceding claim wherein the detection agent is read by a process selected from the group consisting of X-ray film, visual detection of a colorimetric reaction on the membrane, visual detection of a fluorescent tag or by digital imaging.

7. The process of claim 1, wherein the membrane is subjected to said amplification step for from about 30 minutes to about 90 minutes in a temperature controlled device, such as a thermal cycler.

8. The process of claim 1 wherein the detection agent is selected from a fluorescent tag, a radioactive tag, a colorimetric tag and an enzyme tag.

## Patentansprüche

1. Verfahren für das Detektieren einer Mikroorganismenkontamination in einer flüssigen Probe, die aus Wasser, Nahrungsmittelprodukten und Arzneimitteln ausgewählt ist, und das Zählen und Identifizieren dieser etwaigen Mikroorganismen, umfassend die aufeinanderfolgenden Stufen:
a) Filtrieren der flüssigen Probe durch eine zur Retention von Mikroorganismen geeignete Membran, um die Mikroorganismen aus der Probe auf der Membran zurückzubehalten;
c) Durchführen einer Amplifikationsstufe an der Membran, wobei die Amplifikationsstufe ein oder mehrere Detektionsmittel enthält, wobei die Amplifikationsstufe aus der Gruppe von einer Polymerase-Kettenreaktion (PCR), Amplifikation auf Basis der Nukleinsäuresequenz (nucleic acid sequence based amplification, NASBA), Strangverdrängungsamplifikation (strand displacement amplification, SDA) und Linked Linear Amplification (LLA) ausgewählt ist; und
d) Detektieren des Vorhandenseins/Nichtvorhandenseins von einem Mikroorganismus oder mehreren Mikroorganismen, der detektierten Zahl und Art,
**gekennzeichnet durch** die zwischen den Stufen (a) und (c) liegende Stufe
b) Inkubieren der Membran über 1 h bis 4 h zum Erhöhen der auf der Membran zurückgehaltenenen Zahl an Mikroorganismen;
wobei an der Membran in Stufe c) eine Amplifikation über einen Zeitraum von 1 bis 5 h durchgeführt wird.

2. Verfahren nach Anspruch 1, wobei die flüssige Probe aus 50 bis 1000 ml besteht.

3. Verfahren nach Anspruch 1 oder 2, wobei die Membran aus PVDF-Membranen, die durch hydrophobe Abschnitte getrennte hydrophile Bereiche aufweisen, ausgewählt ist.

4. Verfahren nach einem der vorhergehenden Ansprüche, wobei die Amplifikation ein Amplifikationssystem auf Nukleinsäurebasis ist.

5. Verfahren nach Anspruch 4, wobei
a) das Detektionsmittel aus der Gruppe von einer fluoreszenzmarkierten Nukleinsäure, einer Peptid-Nukleinsäuresonde und Gemischen derselben ausgewählt ist und durch ein digitales Bildaufzeichnungssystem erfasst wird, oder
b) die Amplifikation ein Amplifikationssystem auf Nukleinsäurebasis in der Form der Polymerase-Kettenreaktion (PCR) ist und das Detektionsmittel aus der Gruppe von einer fluoreszenzmarkierten Nukleinsäure, einer Peptid-Nukleinsäuresonde und Gemischen derselben ausgewählt ist und durch ein digitales Bildaufzeichnungssystem erfasst wird, oder
c) das Reagenz aus der Gruppe von einem DNA-Target und einem RNA-Target ausgewählt ist und das Detektionsmittel aus der Gruppe von einer fluoreszenzmarkierten Nukleinsäure, einer Peptid-Nukleinsäuresonde und Gemischen derselben ausgewählt ist und durch ein digitales Bildaufzeichnungssystem erfasst wird, oder
d) die Amplifikation ein Amplifikationssystem auf Nukleinsäurebasis in der Form der Polymerase-Kettenreaktion (PCR) ist, das Reagenz aus der Gruppe von einem DNA-Target und einem RNA-Target ausgewählt ist und das Detektionsmittel aus der Gruppe von einer fluoreszenzmarkierten Nukleinsäure, einer Peptid-Nukleinsäuresonde und Gemischen derselben ausgewählt ist und durch ein digitales Bildaufzeichnungssystem erfasst wird.

6. Verfahren nach einem der vorhergehenden Ansprüche, wobei das Detektionsmittel durch ein Verfahren erfasst wird, das aus der Gruppe von einem Röntgenfilm, optischer Detektion einer colorimetrischen Reaktion auf der Membran, optischer Detektion einer fluoreszierenden Markierung oder digitaler Aufzeichnung ausgewählt ist.

7. Verfahren nach Anspruch 1, wobei die Amplifikationsstufe an der Membran über etwa 30 min bis etwa 90 min in einer Vorrichtung mit gesteuerter Temperatur, wie einem Thermocycler, durchgeführt wird.

8. Verfahren nach Anspruch 1, wobei das Detektionsmittel aus einer fluoreszierenden Markierung, einer radioaktiven Markierung, einer colorimetrischen Markierung und einer Enzymmarkierung ausgewählt ist.

## Revendications

1. Procédé de détection d'une contamination par des micro-organismes dans un échantillon liquide choisi parmi de l'eau, des produits alimentaires et des produits pharmaceutiques, ainsi que de numération et d'identification de l'un quelconque parmi de tels micro-organismes, comprenant les étapes séquentielles consistant à :
a) filtrer l'échantillon liquide à travers une membrane appropriée pour la rétention de micro-organismes afin de retenir les micro-organismes provenant dudit échantillon sur ladite membrane ;
c) soumettre la membrane à une étape d'amplification, laquelle étape d'amplification contenant un ou plusieurs agents de détection et étant sélectionnée parmi le groupe constitué de l'amplification en chaîne par polymérase (PCR), l'amplification de séquence d'acides nucléiques (NASBA), l'amplification par déplacement de brin (SDA) et l'amplification linéaire liée (LLA) ; et
d) détecter la présence/l'absence d'un ou plusieurs micro-organismes sur la membrane, leur nombre détecté et leur type,
caractérisé, entre les étapes (a) et (c), par l'étape consistant à :
b) incuber la membrane pendant environ 1 heure à environ 4 heures pour augmenter le nombre de micro-organismes retenues sur ladite membrane, dans lequel la membrane est soumise à une amplification à l'étape C pendant une période d'environ 1 heure à environ 5 heures.

2. Procédé selon la revendication 1, dans lequel l'échantillon liquide représente 50 à 1 000 millilitres.

3. Procédé selon la revendication 1 ou 2, dans lequel la membrane est sélectionnée parmi le groupe constitué des membranes de polyfluorure de vinylidène (PVDF) ayant des zones hydrophiles séparées par des partitions hydrophobes.

4. Procédé selon l'une quelconque des revendications précédentes, dans lequel l'amplification est un système d'amplification fondé sur l'acide nucléique.

5. Procédé selon la revendication 4, dans lequel
a) l'agent de détection est sélectionné parmi le groupe constitué d'un acide nucléique à marquage fluorescent, d'une sonde d'acide nucléique de peptide et de mélanges de ceux-ci, et est lu par un système d'imagerie numérique, ou
b) l'amplification est un système d'amplification fondé sur l'acide nucléique sous la forme d'une amplification en chaîne par polymérase (PCR), et l'agent de détection est sélectionné parmi le groupe constitué d'un acide nucléique à marquage fluorescent, d'une sonde d'acide nucléique de peptide et de mélanges de ceux-ci, et est lu par un système d'imagerie numérique, ou
c) le réactif est sélectionné parmi le groupe constitué d'une cible ADN et d'une cible ARN, et l'agent de détection est sélectionné parmi le groupe constitué d'un acide nucléique à marquage fluorescent, d'une sonde d'acide nucléique de peptide et de mélanges de ceux-ci, et est lu par un système d'imagerie numérique, ou
d) l'amplification est un système d'amplification fondé sur l'acide nucléique sous la forme d'une amplification en chaîne par polymérase (PCR), le réactif étant sélectionné parmi le groupe constitué d'une cible ADN et d'une cible ARN, et l'agent de détection est sélectionné parmi le groupe constitué d'un acide nucléique à marquage fluorescent, d'une sonde d'acide nucléique de peptide et de mélanges de ceux-ci, et est lu par un système d'imagerie numérique.

6. Procédé selon l'une quelconque des revendications précédentes, dans lequel l'agent de détection est lu par un procédé sélectionné parmi le groupe constitué d'un film à rayons X, d'une détection visuelle d'une réaction colorimétrique sur la membrane, d'une détection visuelle d'un indicateur fluorescent ou par imagerie numérique.

7. Procédé selon la revendication 1, dans lequel la membrane est soumise à ladite étape d'amplification pendant environ 30 minutes à environ 90 minutes dans un dispositif à température contrôlée, comme un thermocycleur.

8. Procédé selon la revendication 1, dans lequel l'agent de détection est sélectionné parmi un indicateur fluorescent, un indicateur radioactif, un indicateur colorimétrique et un indicateur d'enzyme.
